**Europäisches Patentamt**

**European Patent Office**

**Office européen des brevets**

(19)

(11) Veröffentlichungsnummer: **0 005 211**
A2

# EUROPÄISCHE PATENTANMELDUNG

(12)

(21) Anmeldenummer: 79101181.0

(22) Anmeldetag: 19.04.79

(51) Int. Cl.²: **C 07 C 109/04**

(30) Priorität: 27.04.78 CH 4585/78

(43) Veröffentlichungstag der Anmeldung: 14.11.79
Patentblatt 79/23

(84) Benannte Vertragsstaaten: **CH DE FR GB IT**

(71) Anmelder: **CIBA-GEIGY AG, Patentabteilung Postfach,
CH-4002 Basel (CH)**

(72) Erfinder: **Somlo, Tibor, Dr., Florastrasse 8, CH-4127
Birsfelden (CH)**
Erfinder: **Vogel, Ulrich, Dr., Liebrütistrasse 44,
CH-4303 Kaiseraugst (CH)**
Erfinder: **Käser, Werner, Dillackerstrasse 26, CH-4142
Münchenstein (CH)**

(54) **Verfahren zur Herstellung von 2,2'-Dichlorhydrazobenzol.**

(57) Verfahren zur Herstellung von 2,2'-Dichlorhydrazobenzol durch Reduktion von 2,2'-Dichlorazoxybenzol mit Formaldehyd und einem Naphthochinon, dadurch gekennzeichnet, dass man die Reduktion in Gegenwart eines nichtionogenen Tensides in einem mit Wasser mischbaren polaren Lösungsmittel durchführt.

- 1 -

3-11 687/=

C I B A - G E I G Y   A G, BASEL

Verfahren zur Herstellung von 2,2'-Dichlorhydrazobenzol

Die deutsche Patentschrift 1 029 005 beschreibt ein Verfahren zur Herstellung von Hydrazobenzolen durch Reduktion der entsprechenden Nitrobenzole in einer ersten Stufe mit Formaldehyd, wässrigem Alkalihydroxyd und einer geringen Menge eines Naphthochinons zum Azoxybenzol und in einer zweiten Stufe mit Formaldehyd in methanolischem Alkalihydroxyd ebenfalls unter Zusatz eines Naphthochinons zum Hydrazobenzol. Gegenüber dem klassischen Reduktionsverfahren mittels Zink hat dieses Verfahren den Vorteil, dass kein die Umwelt belastendes Zinkoxyd anfällt. Während die erste Stufe dieses Verfahrens problemlos verläuft und nahezu quantitative Ausbeuten an Azoxybenzol liefert, beträgt die Ausbeute in der zweiten Stufe an 2,2'-Dichlorhydrazobenzol nur etwa 50 %, wobei 22 % des Ausgangsprodukts zu o-Chloranilin reduziert werden.

- 2 -

Es wurde nun gefunden, dass bei der Herstellung
von 2,2'-Dichlorhydrazobenzol durch Reduktion von 2,2-
Dichlorazoxybenzol mit Formaldehyd und einem Naphthochinon
die Ausbeute in einem technisch einfachen und sicheren Verfahren auf über 80 % mit einer Reinheit von mehr als 90 %
erhöht werden kann, wenn man die Reduktion in Gegenwart
eines nicht-ionogenen Tensids in einem mit Wasser mischbaren polaren Lösungsmittel durchführt. Der Formaldehyd
kann dabei als wässrige Lösung oder als Paraformaldehyd
eingesetzt werden.

Als nichtionogene Tenside kommen vor allem die
folgenden Verbindungen mit den nachstehend angegebenen allgemeinen Formeln in Betracht:

| | |
|---|---|
| Polyalkoholester | $CH_2OH-(CHOH)_n-CH_2COOR$ |
| Polyalkoholäther | $CH_2OH-(CHOH)_n-CH_2OR$ |
| Alkylpolyglykoläther | $R-O-(C_2H_4O)_nH$ |
| Acylpolyglykoläther | $R-COO-(C_2H_4O)_nH$ |
| Alkylarylpolyglykol äther | $R-Ar-O-(C_2H_4O)_nH$ |
| Acylierte Alkanolamin- polyglykoläther | $RCON-[R'-O-(C_2H_4O)_nH]_2$ |
| Alkylierte Alkanol- aminpolyglykoläther | $R-N-[R'-O-(C_2H_4O)_nH]_2$ |
| R und R' bedeuten | Alkylreste mit $C_1 - C_{16}$ |

und n hat einen Wert von 1 bis 20.
Ar                    ist ein aromatischer Ring, insbe-
                      sondere ein Phenylring.

Das nichtionogene Tensid wird in einer Menge von
0,5 bis 10 Gew.%, vorzugsweise von 1 bis 6 Gew.%, bezogen
auf die Menge des zu reduzierenden 2,2'-Dichlorazoxybenzols,
verwendet.

Als mit Wasser mischbare polare Lösungsmittel eignen sich einwertige Alkohole wie Methanol, Aethanol, Propanol, Butanol oder Aethylenglykolmonoalkyläther, von denen Methanol, Aethylenglykolmonomethyläther und Aethylenglykolmonoäthyläther die bevorzugten sind. Als Co-Lösungsmittel können mit Wasser nicht mischbare apolare Lösungsmittel wie beispielsweise aromatische und aliphatische Kohlenwasserstoffe oder chlorierte Kohlenwasserstoffe mitverwendet werden.

Besonders hohe Ausbeuten von über 95 % der Theorie können jedoch erzielt werden, wenn man die Reduktion von 2,2'-Dichlorazoxybenzol zum 2,2'-Dichlorhydrazobenzol in Gegenwart des nichtionogenen Tensids in einem unter den Reaktionsbedingungen beständigen aprotischen polaren Lösungsmittel durchführt. Dazu gehören beispielsweise Acetonitril, Dimethylsulfoxyd, Alkylsulfone und insbesondere Sulfolan. Die Umsetzungstemperatur liegt zweckmässig im Bereich von 10 bis 50°C, vorzugsweise bei 20 bis 30°C, und das als Reduktionsbeschleuniger verwendete Naphthochinon ist vorzugsweise 2,3-Dichlor-1,4-naphthochinon, welches in einer Menge von 0,2 bis 5 Gew.%, vorzugsweise 1 bis 3 Gew.%, bezogen auf das 2,2'-Dichlorazoxybenzol, zugegeben wird.

Das 2,2'-Dichlorhydrazobenzol stellt ein wichtiges Zwischenprodukt für die Herstellung von Farbstoffen und Pigmenten dar, indem es zu 3,3'-Dichlorbenzidin umgelagert und durch Kupplung zum Azofarbstoff verarbeitet wird. Das durch Reduktion mittels Zink hergestellte 2,2'-Dichlorhydrazobenzol ergibt bei der Benzidinumlagerung als Nebenprodukt neben Dichlorbenzidin noch das unerwünschte Monochlorbenzidin. Demgegenüber ergibt das erfindungsgemässe Verfahren ein Dichlorhydrazobenzol, welches sich in monochlorbenzidinfreies Dichlorbenzidin umlagern lässt.

Das erfindungsgemässe Verfahren wird in der Regel so ausgeführt, dass 2,2'-Dichlorazoxybenzol, mit Wasser mischbares polares Lösungsmittel, nichtionogenes Tensid und Naphthochinon vorgelegt und das Gemisch mit 50 %-iger Natronlauge und dann mit Paraformaldehyd versetzt wird. Nach mehrstündiger Reaktionszeit unter Rühren des Gemisches wird das Reaktionsprodukt mit Wasser versetzt, gekühlt, filtriert, gewaschen und getrocknet. Das 2,2'-Dichlorhydrazobenzol kann auch mit Hilfe des Co-Lösungsmittels von der wässrigen Phase abgetrennt und ohne Isolierung auf Dichlorbenzidin verarbeitet werden.Das/zeichnet sich durch besondere Einfachheit und wegen niedriger Rohmaterialkosten auch durch Wirtschaftlichkeit aus und führt in hohen Ausbeuten zu einem 2,2'-Dichlorhydrazobenzol, welches frei von Problem schaffenden Begleitstoffen ist und sich in monochlorbenzidinfreies Dichlorbenzidin umlagern lässt.

In den nachfolgenden Beispielen stellen die Teile Gewichtsteile dar, bezogen auf das Gramm.

B e i s p i e l    1

80 Teile Acetonitril, 80 Teile 2,2'-Dichlorazoxybenzol, 1,8 Teile 2,3-Dichlor-1,4-naphthochinon und 4 Teile eines nicht-ionogenen Emulgators (Tween 20 = Polyoxyäthylenlaurylester der ICI) werden mit 112 Teilen 50 %-iger Natronlauge und darauf bei 25 - 30°C in mehreren Portionen mit 36 Teilen Paraformaldehyd versetzt. Das Gemisch wird noch 4 Stunden bei 30 - 35°C gerührt, 500 ml Wasser zugegeben, auf 10 - 15°C gekühlt, die Kristalle abgenutscht und mit Wasser neutralgewaschen. Bei 70°C im Vakuum getrocknet werden 74,5 g 97,8 %-iges 2,2'-Dichlorhydrazobenzol (= 72,9 g 100 %) mit einem Smp. von 86 - 87°C erhalten. Die Ausbeute entspricht 95,8 % d.Th.

B e i s p i e l   2

80 Teile Sulfolan, 80 Teile 2,2'-Dichlorazoxybenzol, 1,8 Teile 2,3-Dichlor-1,4-naphthochinon und 4 Teile eines nicht-ionogenen Emulgators (Polyoxyäthylen-Laurylalkoholäther der ICI) werden mit 112 Teilen 50 %-iger Natronlauge und darauf bei 25 - 30°C in mehreren Portionen mit 36 Teilen Paraformaldehyd versetzt. Das Gemisch wird noch 4 Stunden bei 30 - 35°C gerührt, dann mit 500 ml Wasser versetzt und auf 10 - 15°C gekühlt. Die ausgefallenen Kristalle werden darauf abgenutscht, mit Wasser neutral gewaschen, mit Wasser nochmals ausgekocht, abgekühlt, filtriert und bei 70°C im Vakuum getrocknet. Es werden 75,3 g reines 2,2'-Dichlorhydrazobenzol, entsprechend 99 % d.Th., mit einem Smp. von 88°C erhalten. Gehalt des Produktes über 99 %.

B e i s p i e l   3

Werden 80 Teile Dimethylsulfoxyd anstatt Sulfolan eingesetzt, so werden 74,3 g 96,5 %-iges 2,2'-Dichlorhydrazobenzol, entsprechend 94,4 % der Theorie, erhalten.

B e i s p i e l   4

80 Teile Methanol, 80 Teile 2,2'-Dichlorazoxybenzol, 1,8 Teile 2,3-Dichlor-1,4-naphthochinon und 4 Teile eines nicht ionogenen Emulgators (Tween 20) werden bei 25 - 30°C mit 112 Teilen 50 %-iger Natronlauge und darauf ebenfalls bei 25 - 30°C in mehreren Portionen mit 36 Teilen
Paraformaldehyd versetzt. Das Gemisch wird noch 4 Stunden
bei 25 - 30°C nachgerührt, dann mit 500 ml Wasser verdünnt
und auf 10 - 15°C gekühlt, die ausgefallenen Kristalle werden abgenutscht und mit ca. 1000 ml Wasser neutralgewaschen.
Bei 60 - 70°C im Vakuum getrocknet werden 68,5 g 2,2'-Dichlorhydrazobenzol 95 % (= 65 g 100 %), entsprechend
85,6 % d.Th., erhalten.


B e i s p i e l   5


200 Teile 2,2'-Dichlorazoxybenzol in 200 Teilen
Solventnaphtha gelöst (die Lösung kann aus der vorhergehenden Reduktionsstufe stammen) werden mit 50 Teilen
Methanol, 4,5 Teilen 2,3-Dichlor-1,4-naphthochinon und
10 Teilen eines nicht-ionogenen Emulgators (Tween SD 20
= Polyoxyäthylen-sorbitan-laurylester der ICI) versetzt.
Dazu werden bei 25 - 30°C 280 Teile 50 %-iger Natronlauge
und anschliessend innerhalb 2 Stunden bei 25 - 30°C 90 Teile Paraformaldehyd gegeben. Nach 4 Stunden Nachreaktion
bei 25 - 30°C werden 285 Teile Solventnaphtha und 325 Teile Wasser zugegeben, 5 Minuten gerührt und dann die Schichten getrennt. Die Solventnaphtha-Schicht enthält 165 g
2,2'-Dichlorhydrazobenzol, was einer Ausbeute von 87 % d.Th.
entspricht. Das Produkt kann in der Solventnaphthalösung
auf 3,3'-Dichlorbenzidin verarbeitet werden.


B e i s p i e l   6


80 Teile Aethylcellosolve (Aethylenglykolmonoäthyläther), 80 Teile 2,2'-Dichlorazoxybenzol, 1,8 Teile
2,3-Dichlor-1,4-naphthochinon und 4 Teile Tween 20 werden
bei 25 - 30°C mit 112 Teilen 50 %-iger Natronlauge und darauf mit 36 Teilen Paraformaldehyd in mehreren Portionen versetzt. Das Gemisch wird bei 25 - 30°C 4 Stunden nachgerührt und mit 500 ml Wasser verdünnt, die ausgefallenen Kristalle werden abgenutscht und mit ca. 1000 ml Wasser neutralgewaschen. Im Vakuum getrocknet erhält man 69,6 g 2,2'-Dichlorhydrazobenzol 95 %, was einer Ausbeute von 87,1 % d.Th. entspricht.

B e i s p i e l    7

Werden anstatt 80 Teile Aethylcellosolve des Beispiels 3  80 Teile Methylcellosolve eingesetzt, so werden 69,9 g 2,2'-Dichlorhydrazobenzol 95 %, entsprechend 87,4 % d.Th., erhalten.

Patentansprüche

1.     Verfahren zur Herstellung von 2,2'-Dichlorhydrazobenzol durch Reduktion von 2,2'-Dichlorazoxybenzol mit
Formaldehyd und einem Naphthochinon, dadurch gekennzeichnet,
dass man die Reduktion in Gegenwart eines nichtionogenen
Tensides in einem mit Wasser mischbaren polaren Lösungsmittel durchführt.

2.     Verfahren gemäss Anspruch 1, dadurch gekennzeichnet, dass man als nichtionogenes Tensid einen Polyalkoholester, Polyalkoholäther, Alkylpolyglykoläther, Acylpolyglykoläther, Alkylarylpolyglykoläther oder acylierten oder
alkylierten Alkanolaminpolyglykoläther verwendet.

3.     Verfahren gemäss Anspruch 1, dadurch gekennzeichnet, dass man die Reduktion in einem unter den Reaktionsbedingungen inerten aprotischen polaren Lösungsmittel durchführt.

4.     Verfahren gemäss Anspruch 1, dadurch gekennzeichnet, dass man die Reduktion in einem einwertigen Alkohol
durchführt.

5.     Verfahren gemäss Anspruch 4, dadurch gekennzeichnet, dass man ein mit Wasser nicht mischbares apolares Lösungsmittel mitverwendet.

6.     Verfahren gemäss Anspruch 1, dadurch gekennzeichnet, dass man als Naphthochinon 2,3-Dichlor-1,4-naphthochinon verwendet.

7.      Verfahren gemäss Anspruch 1, dadurch gekennzeichnet, dass man die Reduktion im Temperaturbereich von 10 bis 50°C durchführt.